**Europäisches Patentamt**

**European Patent Office**

**Office européen des brevets**

(11) Publication number: **0 129 610**
**B1**

# EUROPEAN PATENT SPECIFICATION

(45) Date of publication of patent specification: **06.05.87**

(21) Application number: **83106095.9**

(22) Date of filing: **22.06.83**

(51) Int. Cl.⁴: **C 07 D 473/12, A 23 F 5/22**

(54) Regeneration of caffeine-loaded activated carbon with hot water.

(43) Date of publication of application:
**02.01.85 Bulletin 85/01**

(45) Publication of the grant of the patent:
**06.05.87 Bulletin 87/19**

(84) Designated Contracting States:
**AT BE CH FR IT LI NL**

(56) References cited:
**EP-A-0 042 295**
**EP-A-0 076 620**
**EP-A-0 078 088**
**FR-A-2 408 607**

(73) Proprietor: **HAG GF AKTIENGESELLSCHAFT**
**Hagstrasse**
**D-2800 Bremen 1 (DE)**

(72) Inventor: **Gehrig, Manfred, Dr.**
**Lug-ins-Land 10**
**D-8069 Wolnzach (DE)**
Inventor: **Barthels, Manfred**
**Elsässer Strasse 70**
**D-2800 Bremen (DE)**
Inventor: **Wienges, Hans R.**
**Helene-Neesen-Strasse 8**
**D-2800 Bremen (DE)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Courier Press, Leamington Spa, England.

## Description

Technical Field

The invention relates to a process for recovering caffeine adsorbed to activated carbon with simultaneous regeneration of the activated carbon in which the caffeine-loaded activated carbon is treated with water at a pressure of at least 86 bar and at a temperature of at least 300°C.

Background Art

For health reasons the decaffeination of vegetable products has gained considerable significance. Moreover, the caffeine obtained as by-product can be profitably sold.

In several decaffeination processes the hydrolyzed coffee beans are decaffeinated by means of organic solvents. The recovery of the solvent is effected by distillation, for example, in the course of which the caffeine is obtained as by-product.

In order to avoid any contamination of the vegetable material by solvent residues, carbon dioxide has recently been used as extractant as it is unobjectionable under the health aspect (German Patents Nos. 2,005,293 and 2,212,281). The carbon dioxide solvent in these processes is freed from dissolved caffeine by means of activated carbon.

Prior to re-use of spent activated carbon the adsorbed substances are normally pyrolyzed and thereafter the carbon is thermally reactivated. On account of the usefulness of caffeine such a method is uneconomical.

Therefore, efforts have been made to recover the adsorbed caffeine. In the selection of measures for desorption of caffeine it must be borne in mind that active carbon is a very good adsorbent, a circumstance which renders desorption difficult. Moreover, the use of any agent objectionable under the health aspect is to be avoided since the extraction was carried out with carbon dioxide for the very reason to exclude such agents. For efficient and economical regeneration of the activated carbon the solvent should have a high dissolving capacity for the adsorbed substance and good transporting properties for the substance; thereafter the mixture should be readily separable.

According to the teaching of German OS 2,544,116 the adsorbate is desorbed with supercritical gases, especially with carbon dioxide. Thereafter the dissolved adsorbate must be removed from the dissolving gas.

A process is known from EP—A—78,088 in which among other things caffeine is bound to an adsorbent which is subsequently regenerated with the aid of water or a mixture of water and alcohol. Practically no statements are made about temperature and pressure conditions during the regeneration.

A similar process is also known from French Patent 2,408,607. There, too, no statements are made about the pressure conditions during the regeneration of the caffeine-loaded activated carbon. The point here is that the regeneration takes place at normal pressure. The temperature during the regeneration is between 50 and 100°C.

U.S. Patent No. 4,298,736 describes a process in which caffeine adsorbed to activated carbon is desorbed with a food-grade liquid solvent which may be an organic acid or an alcohol. The process is preferably carried out above 100°C with glacial acetic acid or with azeotropic mixtures of glacial acetic acid and second components.

After regeneration of the activated carbon with non-volatile solvents the solvent must be separated from the activated carbon in any event with steam, for example. This necessarily calls for an additional step.

It is the problem underlying the invention to provide a simple and economical process for recovering caffeine adsorbed to activated carbon with simultaneous regeneration of the activated carbon; the desorption of caffeine from the activated carbon is to be effected with a food-grade agent.

Disclosure of the Invention

This problem is solved by a process in which the desorption of the caffeine from the activated carbon is carried out with simultaneous regeneration of the activated carbon with water at a pressure of at least 86 bar, preferably at least 150 bar, and at a temperature of at least 300°C.

Caffeine has been known to be water-soluble up to a certain degree (the solubility is 1.3% at 15°C and 4.6% at 40°C); however, tests with hot water revealed that the desorption of caffeine from the activated carbon is virtually impossible under these conditions. With the present invention it has been surprisingly found that the activated carbon can be substantially completely freed from caffeine and the caffeine can be simultaneously recovered by means of water at a pressure of at least 86 bar, preferably at least 150 bar, and at a temperature of at least 300°C. A preferred temperature in carrying out the process is about 350°C, while a preferred pressure is about 200 bar. The period of treating the activated carbon ranges preferably from about 1 to 3 hours: for desorption of caffeine with simultaneous regeneration of the activated carbon preferably at least 15 times, especially about 20 times the amount of water, based on the weight of the caffeine-loaded activated carbon, should be used.

After sufficient desorption of caffeine, the temperature is raised to 350°C in a preferred embodiment of the present process to decompose the residual caffeine.

In a further preferred embodiment of the present process, the caffeine is recovered from the aqueous solution by evaporation, concentration by membranes, freezing, salting out, or by extraction with an organic solvent.

The individual measures such as pressure, temperature, amount of water and treating period can be easily selected by the expert from case to case in optimum correlation. It only need be considered that, although under more rigid conditions, i.e., excessively high pressure and temperature conditions and longer treating periods, the desorption of all the caffeine from the activated carbon is possible, this brings about the risk of excessive decomposition of the caffeine due to hydrolysis and/or thermal degradation.

The process of the invention can be carried out batch-wise or continuously.

The activated carbon obtained with the process of the invention can be re-used immediately without requiring any additional treating step. The adsorption activity of the activated carbon diminishes only slightly from one recycle to the next. High temperature reactivation is required only after many times of recycling the activated carbon.

In the following example the below-described mode of operation was adopted:

Water heated to the desired temperature in an autoclave was forced by a pump through a bed of activated carbon to be subjected to extraction. The activated carbon was contained in a heatable steel tube. A regulating valve served as throttle and maintained a pressure which prevented vaporization of the water in the autoclave. Downstream of the throttle the solution was cooled and trapped in fractions.

Example

50 grams of loaded activated carbon having a water content of 5.87% and caffeine proportion of 8.3% in dry condition were extracted at 345°C and at 200 bar with 20 times the amount of water. The test lasted for 150 minutes. 75% of the adsorbed caffeine were found in the solution (0.29%); only 3.4% of the adsorbed caffeine remained on the activated carbon. After a period of 75 minutes and after a throughput of 10 times the amount of water 64% of the adsorbed caffeine had been extracted.

Claims

1. A process for recovering caffeine adsorbed to activated carbon with simultaneous regeneration of the activated carbon, characterized in that the caffeine-loaded activated carbon is treated with water at a pressure of at least 86 bar and at a temperature of at least 300°C.

2. Process according to claim 1 in which, after sufficient desorption of caffeine, the temperature is raised to 350°C to decompose the residual caffeine.

3. Process according to claim 1 in which the caffeine is recovered from the aqueous solution by evaporation, concentration by membranes, freezing, salting out, or by extraction with an organic solvent.

**Patentansprüche**

1. Verfahren zur Wiedergewinnung von auf Aktivkohle adsorbiertem Coffein unter gleichzeitiger Regenerierung der Aktivkohle, dadurch gekennzeichnet, daß man die mit Coffein beladene Aktivkohle bei einem Druck von wenigstens 86 bar und einer Temperatur von wenigstens 300°C mit Wasser behandelt.

2. Verfahren nach Anspruch 1, bei dem nach ausreichender Desorption von Coffein die Temperatur auf über 350°C zur Zersetzung des restlichen Coffeins erhöht wird.

3. Verfahren nach Anspruch 1, bei dem das Coffein durch Eindampfen, Konzentrieren durch Membranen, Ausfrieren, Aussalzen oder durch Extraktion mit einem organischen Lösungsmittel aus der wässrigen Lösung gewonnen wird.

**Revendications**

1. Un procédé pour récupérer la caféine adsorbée sur du charbon activé avec régénération simultanée du charbon activé, caractérisé en ce que l'on traite le charbon activé chargé de caféine, par de l'eau à une pression d'au moins 86 bars et à une température d'au moins 300°C.

2. Un procédé selon la revendication 1, dans lequel, après désorption suffisante de la caféine, on élève la température à 350°C pour décomposer la caféine résiduelle.

3. Un procédé selon la revendication 1, dans lequel on récupère la caféine dans la solution aqueuse par évaporation, concentration au moyen de membranes, congélation, relargage, ou extraction par un solvant organique.